(19) Europäisches Patentamt
European Patent Office
Office européen des brevets

(11) Numéro de publication: **0 069 013**
**A1**

(12) **DEMANDE DE BREVET EUROPEEN**

(21) Numéro de dépôt: **82401155.5**

(22) Date de dépôt: **23.06.82**

(51) Int. Cl.³: **C 07 D 307/16**, C 07 B 19/00

(30) Priorité: **30.06.81 FR 8113061**

(43) Date de publication de la demande: **05.01.83**
**Bulletin 83/1**

(84) Etats contractants désignés: **AT BE CH DE FR GB IT LI LU NL SE**

(71) Demandeur: **SANOFI, Société dite:, 40, Avenue George V, F-75008 Paris (FR)**

(72) Inventeur: **Biasioli, Carlo, 176 Avenue de Muret, F-31100 Toulouse (FR)**
Inventeur: **Heymes, Alain, Chemin de l'Adrech, F-04200 Sisteron (FR)**

(74) Mandataire: **Polus, Camille et al, c/o Cabinet Lavoix 2, Place d'Estienne d'Orves, F-75441 Paris Cedex 09 (FR)**

(54) **Isomères de naftidrofuryl et procédé pour leur préparation.**

(57) L'invention a pour objet les Diasteroisoméres Ad1 et Bd1 et les Enantioméres Bd et B1 du Naftidrofuryl, ainsi que leurs sels et leur procédé de séparation et d'isolement.

EP 0 069 013 A1

ACTORUM AG

0069013

# Isomères de NAFTIDROFURYL et procédé pour leur préparation.-

Le NAFTIDROFURYL ou (naphtyl-1) - 3 tétrahydrofurfuryl-2 propionate de N-diéthylamino-2 éthyle est connu, en thérapeutique humaine, principalement pour ses propriétés vasodilatatrices périphériques. Il répond à la formule développée suivante :

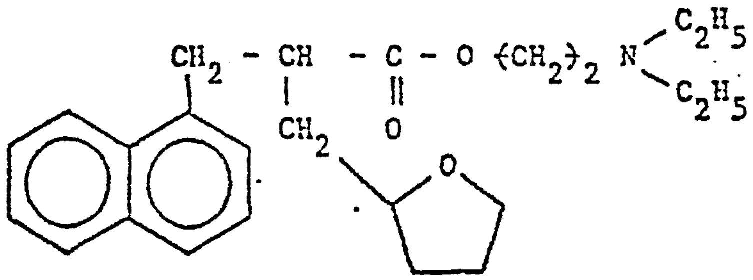

Néanmoins, en raison de la présence de deux carbones asymétriques, cette molécule recouvre deux composés isomères qualifiés de DIASTEREOISOMERES (A et B) eux-mêmes dédoublables en deux antipodes optiques ou ENANTIOMERES, l'un dextrogyre, l'autre lévogyre (d et l).

Les analyses réalisées sur un grand nombre d'échantillons de NAFTIDROFURYL du commerce, qui ne présente aucune activité optique, ont montré que le rapport des DIASTEREOISOMERES Adl et Bdl était variable et qu'il se situait entre 28/72 et 42/58, avec une valeur moyenne de 35/65. Or, ces variations sont incompatibles avec les réglementations pharmaceutiques en vigueur qui stipulent que les produits utilisés en thérapeutique humaine doivent répondre à des critères de pureté fixes d'où sont déduites les propriétés et les utilisations en cause.

La présente invention a donc pour objet les DIASTEREOisomères Adl et Bdl et les ENANTIOMERES Bd et Bl du NAFTIDROFURYL et leur procédé de séparation et d'isolement.

La séparation et l'isolement des DIASTEREOISOMERES Adl et Bdl est réalisée en opérant des recristallisations fractionnées sur des mélanges de A (dl) et B (dl) sous forme de différents sels tels qu'oxalate, nitrate, citrate ou méthanesulfonate en travaillant dans chaque cas dans un solvant ou un mélange de solvant bien approprié.

Il est bien entendu possible, après isolement des DIASTEREOISOMERES Adl et Bdl sous forme d'un des sels précités,

de changer la nature de ce sel pour un autre sel tel que décrit ou tout autre sel pharmaceutiquement acceptable, en opérant par des moyens connus une libération de la base et une salification de cette dernière.

La séparation et l'isolement des ENANTIOMERES B (d) et B (l) sont obtenus par la séquence réactionnelle suivante :

a) on sépare le mélange diastéréoisomère Bdl sous forme de l'un de ses sels selon le procédé décrit ci-dessus ;

b) on saponifie dans des conditions non épimérisantes le produit de séparation de l'étape précédente pour obtenir le mélange d'acides (naphtyl-1)-3 tétrahydrofurfuryl-2 propionique B(dl) sous forme cristalline ;

c) on salifie le mélange d'acides B(dl) obtenu avec une amine optiquement active ;

d) on dédouble le sel d'amine obtenu par recristallisations successives dans des solvants appropriés ;

e) on régénère respectivement les acides B(d) et B(l) dédoublés et on les recristallise dans un solvant approprié ;

f) on estérifie les acides B(d) et B(l) respectifs avec le N-diéthylamino-2 chloro-1 éthane dans des conditions non épimérisantes ; et

g) on salifie les énantiomères B(d) et B(l) obtenus avec des acides pharmaceutiquement acceptables.

Un acide pharmaceutiquement acceptable est, par exemple, l'acide citrique.

L'amine optiquement active est notamment l'α-méthyl benzylamine (d) ou (l).

L'ensemble de ces opérations peut être schématisé comme suit :

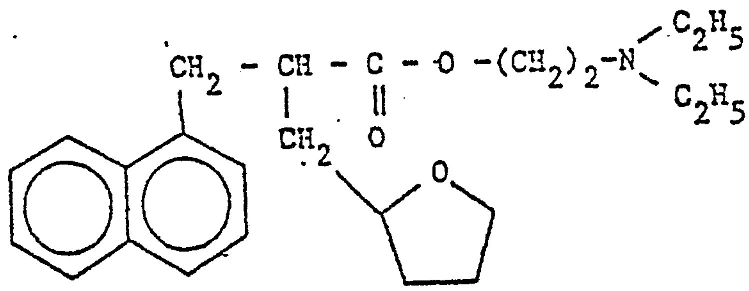

DIASTEREOISOMERE B (dl)

saponification

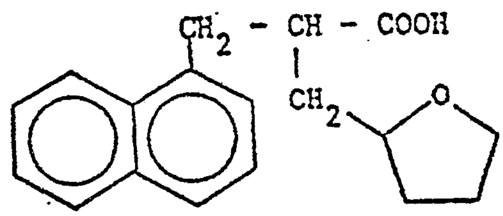

Acide B (dl)

recristallisations des sels avec Ø-$CH-NH_2$ (d)
$CH_3$
régénération et recristallisation

Acide B (d)

estérification

ENANTIOMERE B (d)

salification

Citrate de l'ENANTIOMERE B (d)

recristallisations des sels Ø-$CH-NH_2$ (l)
$CH_3$
régénération et recristallisation

Acide B (l)

estérification

ENANTIOMERE B (l)

salification

Citrate de l'ENANTIOMERE B (l)

Il est décrit, ci-dessous, des exemples non limitatifs de séparation des DIASTEREOISOMERES A et B et des ENANTIOMERES Bd et Bl.

EXEMPLE 1

Séparation et dosage, par chromatographie liquide haute pression, des deux diastéréoisomères A (dl) et B (dl) du Naftidrofuryl

Colonne :silice 5 µ (Partisil PXS 5/25 Whatman)
Phase mobile :isooctane/dichlorométhane/méthanol/triéthylamine 90/9,5/0,5/0,1.
Débit :1,5 ml/mn
Détection :U.V. à 280 nm

Le diastéréoisomère A (dl) a un temps d'élution d'environ 19 minutes et le diastéréoisomère B (dl) d'environ 21 minutes.

EXEMPLE 2

Séparation et isolement de l'oxalate du DIASTEREOISOMERE B (dl) de Naftidrofuryl

160 grammes d'oxalate de NAFTIDROFURYL (F = 100°C), dont le rapport des DIASTEREOISOMERES A et B est de 30/70, sont recristallisés dans 8 l d'acétone pour fournir après filtration et séchage 113,3 grammes d'un mélange diastéréoisomérique 17/83, F = 110°C, qui recristallisés dans 6,38 l d'acétone conduisent à 55 g d'un mélange diastéréoisomérique 7/93, F = 100°C, qui finalement recristallisés dans 3,4 l d'acétone fournissent 34,5 g de l'oxalate du DIASTEREOISOMERE B pur de Naftidrofuryl, F = 110°C.

Analyse : $C_{24}H_{33}NO_3$, $C_2H_2O_4$ = 473,5

| | | | | |
|---|---|---|---|---|
| . calculé : | C % 65,94 | H % 7,45 | N % 2,96 |
| . trouvé : | 66,04 | 7,53 | 2,95 |

EXEMPLE 3

Séparation et isolement du nitrate du DIASTEREOISOMERE B (dl) de Naftidrofuryl

8,6 kg de nitrate de NAFTIDROFURYL, F = 120°C, dont le rapport des DIASTEREOISOMERES A et B est de 35/65, recristallisés dans 40 l d'éthanol, fournissent après filtration et séchage 7,5 kg d'un mélange diastéréoisomérique 15/85, F = 120°C. Une seconde recristallisation conduit à 6,2 kg d'un mélange diastéréoisomérique 11/89, une troisième à 5,45 kg d'un mélange diastéréoisomérique 7/93, une quatrième à 4,2 kg d'un mélange diastéréoisomérique 5/95 et finalement une cinquième recristallisation fournit 3,8 kg de nitrate du DIASTEREOISOMERE B pur de Naftidrofuryl, F = 123°C.

Analyse : $C_{24}H_{33}NO_3$, $HNO_3$ = 446,5

. càlculé : C % 64,55 H % 7,67 N % 6,27

. trouvé : 64,39 7,62 6,33

EXEMPLE 4

Séparation et isolement de l'oxalate du DIASTEREOISOMERE A (dl) de Naftidrofuryl

164 grammes de NAFTIDROFURYL oxalate, facilement accessibles à partir des eaux-mères des recristallisations opérées tel qu'à l'exemple 2 décrit ci-dessus, recristallisés dans 5,5 l d'un mélange 8-2 d'acétate d'éthyle et d'isopropanol fournissent 86,3 g d'un mélange diastéréoisomérique 88/12 d'oxalate de NAFTIDROFURYL.

Une nouvelle recristallisation dans 2,85 l du même mélange conduit à 53 g d'un mélange diastéréoisomérique 91/9 d'oxalate de NAFTIDROFURYL. Une troisième recristallisation dans un mélange 9-1 des mêmes solvants fournit 17 g d'oxalate de DIASTEREOISOMERE A de Naftidrofuryl, F = 108°C.

Analyse : $C_{24}H_{33}NO_3$, $C_2H_2O_4$ = 473,5

. calculé : C % 65,94 H % 7,45 N % 2,96

. trouvé : 66,11 7,61 2,92

EXEMPLE 5

Séparation et isolement du Méthanesulfonate du DIASTEREOISOMERE B (dl) de Naftidrofuryl

A une solution de 5 g (1,3 . $10^{-2}$ mole) de DIASTEREOISOMERE B (dl) base dans un mélange de 50 ml d'éther isopropylique et de 15 ml d'alcool isopropylique, on additionne 1,25 g (1.3.$10^{-2}$ mole) d'acide méthanesulfonique. Après chauffage au reflux et refroidissement lent du milieu, le précipité formé est filtré et séché.

On obtient ainsi 5,1 g de méthanesulfonate de DIASTEREOISOMERE B (dl) de Naftidrofuryl, F = 94° C.

Analyse : $C_{24}H_{33}NO_3$, $CH_4O_3S$ = 479,5

. calculé : C % 62,60 H % 7,78 N % 2,92

. trouvé : 62,37 7,70 2,73

EXEMPLE 6

Séparation et isolement du Citrate du DIASTEREOISOMERE B (dl) de Naftidrofuryl

A une solution de 50 g (0,13 mole) du DIASTEREOISOMERE B (dl) de Naftidrofuryl base dans un mélange de 250 ml d'acétone et 250 ml d'acétate d'éthyle, on additionne 27,4 g (0,13 mole) d'acide citrique monohydraté. Le milieu réactionnel est porté au reflux puis refroidi à 5°C. Le précipité formé, filtré et séché fournit 64 g de citrate de DIASTEREOISOMERE B (dl) de Naftidrofuryl, F = 78°C.

Analyse : $C_{24}H_{33}NO_3$, $C_6H_8O_7$ = 583,5

. calculé : C % 62,59 H % 7,18 N % 2,43

. trouvé : 62,01 7,13 2,48

EXEMPLE 7

Citrate de (Naphtyl-1)-3 tétrahydrofurfuryl-2 propionate de N-diéthylamino-2 éthyle B (l)

a) Acide (Naphtyl-1)-3 tétrahydrofurfuryl-2 propionique B (dl)

600 g de nitrate du DIASTEREOISOMERE B (dl) de Naftidrofuryl préparés selon le procédé décrit à l'exemple 3, sont additionnés successivement de 2 l d'éther isopropylique et de 700 ml d'une solution aqueuse environ 3 N d'hydroxyde de sodium. On isole, après décantation, la phase organique, réextrait la phase aqueuse avec 0,2 l d'éther isopropylique puis réunit les phases éthérées. Après séchage sur sulfate de sodium puis évaporation du solvant, on obtient 510 g du DIASTEREOISOMERE B (dl) de Naftidrofuryl base sous forme d'une huile que l'on met à réagir durant 48 heures à température ambiante dans un mélange de 1 l de méthanol et de 1 l d'une solution aqueuse 2 N d'hydroxyde de sodium. Après ajustement à 6 du pH par addition d'acide chlorhydrique aqueux on évapore sous vide le méthanol. Le milieur réactionnel est alcalinisé par addition d'une solution aqueuse 2 N d'hydroxyde de sodium et extrait avec de l'éther isopropylique. La phase aqueuse isolée est

acidifiée de nouveau à l'aide d'acide chlorhydrique aqueux puis extraite avec de l'éther isopropylique. Après séchage sur sulfate de sodium et évaporation de cette phase organique on obtient 320 g d'acide (naphtyl-1)-3 tétrahydrofurfuryl-2 propionique B (dl) sous forme de cristaux blancs, F = 90°C. Une analyse par chromatographie liquide haute-pression répondant aux caractéristiques suivantes, permet de contrôler la pureté isomérique (absence d'acide A (dl) du produit préparé) :

| | | |
|---|---|---|
| Colonne | : | silice 5 µ (Partisil PXS 5/25 Whatman) |
| Phase mobile | : | isooctane/éther isopropylique/dichloro-1,2 éthane/méthanol/acide acétique : 80 / 10 / 10 / 0,5 / 0,1. |
| Débit | : | 1,0 ml/mn |
| Détection | : | U.V. à 280 nm |

L'acide A (dl) a un temps d'élution d'environ 24 minutes et l'acide diastéréoisomère B (dl) d'environ 26 minutes.

b) Acide (Naphtyl-1)-3 tétrahydrofurfuryl-2 propionique B (l)

Un mélange de 150 g d'acide (Naphtyl-1)-3 tétrahydrofurfuryl-2 propionique B (dl) obtenu tel que décrit ci-dessus, 69 ml d'alpha-méthylbenzylamine l, 750 ml d'éther isopropylique et 100 ml d'alcool éthylique est porté au reflux. La solution obtenue laisse précipiter par refroidissement des cristaux que l'on filtre puis recristallise plusieurs fois dans l'éther isopropylique. Par les moyens habituels, on régénère l'acide dédoublé de son sel et obtient après recristallisation dans l'éther isopropylique 30 g d'acide (naphtyl-1)-3 tétrahydrofurfuryl-2 propionique B (l).

$$[\alpha]^{25°C}_{589\ nm} = -34,7° \text{ (méthanol)}$$

L'excès énantiomérique $ee = \frac{|\%\ l - \%\ d|}{100}$ est supérieur à 95 %

Cet excès est déterminé en préparant par les moyens habituels l'amide de l'acide obtenu et de l'alpha-méthylbenzylamine l et en mesurant par chromatographie liquide haute pression, dont les caractéristiques sont indiquées ci-après, les pourcentages respectifs des deux amides diastéréoisomères obtenus :

Colonne : silice 5 µ (Partisil PXS 5/25 Whatman)

Phase mobile : Isooctane/dichloro-1,2 éthane/éther isopropylique/méthanol/eau : 80 / 10 10 / 1 / 0,02.

Débit : 1,3 ml/mn

Détection : U.V. à 280 nm

Les deux amides diastéréoisomères ont des temps d'élution respectivement de 19,5 mn et 25,5 mn.

c) Citrate de (naphtyl-1)-3 tétrahydrofurfuryl-2 propionate de N-diéthyl-aminoéthyle B (l)

Un mélange de l'acide obtenu ci-dessus (29 g), 20 g de N-diéthylamino-2 chloro-1 éthane, 6,2 g d'hydroxyde de sodium en pastilles et 100 ml d'isopropanol est porté au reflux durant 8 heures. Après évaporation du solvant sous vide et traitements habituels on obtient 30 g de (naphtyl-1)-3 tétrahydrofurfuryl-2 propionate de N-diéthyl-aminoéthyle B (l) qui sont salifiés tel que décrit à l'exemple 6 pour donner 33 g de citrate de (naphtyl-1)-3 tétrahydrofurfuryl-2 propionate de N-diéthyl aminoéthyle B(l).

$[\alpha]^{20,5°}_{589\ nm}$ = -49° (méthanol)

F = 91° C

Analyse : $C_{24}H_{33}NO_3$, $C_6H_8O_7$ : 583,5

. calculé : C % 62,59 H % 7,18 N % 2,43

. trouvé : 62,63 7,18 2,58

EXEMPLE 8

Citrate de (naphtyl-1)-3 tétrahydrofurfuryl-2 propionate de N-diéthylamino-2 éthyle B (d)

On procède comme à l'exemple 7 à partir de 150 g d'acide (naphtyl-1)-3 tétrahydrofurfuryl-2 propionique B (dl) mais en mettant en oeuvre l'alphaméthylbenzylamine (d).

a) Acide (naphtyl-1)-3 tétrahydrofurfuryl-2 propionique B (d)

. m = 27,3 g

. $[\alpha]^{25°C}_{589\ nm}$ = + 28° (méthanol)

. ee = 74 % (Cf exemple 7)

b) Citrate de (naphtyl-1)-3 tétrahydrofurfuryl-2 propionate de N-diéthylamino-2 éthyle B (d)

. m = 32 g

. F = 91°C

. $[\alpha]_{589\ nm}^{20,5°C}$ = + 40,7° (méthanol)

Analyse : $C_{24}H_{33}NO_3$, $C_6H_8O_7$ = 583,5

| | | | | | | | |
|---|---|---|---|---|---|---|---|
| . calculé : | C % | 62,59 | H % | 7,18 | N % | 2,43 |
| . trouvé : | | 62,51 | | 7,30 | | 2,40 |

## REVENDICATIONS

1. Diastéréoisomères et énantiomères du Naftidrofuryl.

2. Diastéréoisomère Adl suivant la revendication 1, caractérisé par le point de fusion de son oxalate qui est de 108°C.

3. Diastéréoisomère Bdl suivant la revendication 1, caractérisé par le point de fusion de son oxalate qui est de 110°C.

4. Enantiomère Bd suivant la revendication 1, caractérisé par le point de fusion de son citrate qui est de 91°C.

5. Enantiomère Bl suivant la revendication1, caractérisé par le point de fusion de son citrate qui est de 91°C.

6. Procédé de préparation des diastéréoisomères Adl et Bdl de Naftidrofuryl, caractérisé en ce qu'on soumet le mélange de diastéréoisomères à une séparation par chromatographie liquide haute pression.

7. Procédé de préparation des diastéréoisomères Adl et Bdl de Naftidrofuryl, caractérisé en ce qu'on soumet un mélange de diastéréoisomères de Naftidrofuryl sous forme de sels à des recristallisations successives dans un solvant ou un mélange de solvants appropriés.

8. Procédé selon la revendication 7, caractérisé en ce que les sels sont choisis parmi l'oxalate, le nitrate, le citrate, le méthanesulfonate ou tout autre sel pharmaceutiquement acceptable.

9. Procédé selon la revendication 7 ou 8, caractérisé en ce que le solvant est choisi parmi l'acétone, l'éthanol, l'isopropanol, l'éther isopropylique ou leurs mélanges.

10. Procédé de préparation des énantiomères B(d) et B(l) du Naftidrofuryl, caractérisé en ce que :

a) on sépare le mélange diastéréoisomère Bdl sous forme de l'un de ses sels selon le procédé décrit dans l'une quelconque des revendications 6 à 9;

b) on saponifie dans des conditions non épimérisantes le produit de séparation de l'étape précédente pour obtenir le mélange d'acides (naphtyl-1)-3 tétrahydrofurfuryl-2 propionique B(dl) sous forme cristalline ;

c) on salifie le mélange d'acides B(dl) obtenu avec une amine optiquement active;

d) on dédouble le sel d'amine obtenu par recristallisations successives dans des solvants appropriés;

e) on régénère respectivement les acides B(d) et B(l) dédoublés et on les recristallise dans un solvant approprié;

f) on estérifie les acides B(d) et B(l) respectifs avec le N-diéthylamino-2 chloro-1 éthane dans des conditions non épimérisantes; et

g) on salifie les énantiomères B(d) et B(l) obtenus avec des acides pharmaceutiquement acceptables.

11. Procédé suivant la revendication 10, caractérisé en ce que l'amine optiquement active est l'α-méthyl-benzylamine (d) ou (l) respectivement.

12. Procédé suivant la revendication 10, caractérisé en ce que le solvant de recristallisation de l'étape (d) est l'éther isopropylique.

13. Procédé suivant la revendication 10, caractérisé en ce que l'acide utilise dans l'étape (g) est choisi parmi l'acide oxalique, citrique, nitrique, méthanesulfonique.

AUTRICHE

## REVENDICATIONS

1 - Procédé de préparation des diastéréoisomères Adl et Bdl de Naftidrofuryl, caractérisé en ce qu'on soumet le mélange de diastéréoisomères à une séparation par chromatographie liquide haute pression.

2 - Procédé de préparation des diastéréoisomères Adl et Bdl de Naftidrofuryl, caractérisé en ce qu'on soumet un mélange de diastéréoisomères de Naftidrofuryl sous forme de sels à des recristallisations successives dans un solvant ou un mélange de solvants appropriés.

3 - Procédé selon la revendication 2, caractérisé en ce que les sels sont choisis parmi l'oxalate, le nitrate, le citrate, le méthanesulfonate ou tout autre sel pharmaceutiquement acceptable.

4 - Procédé selon les revendications 2 ou 3, caractérisé en ce que le solvant est choisi parmi l'acétone, l'éthanol, l'isopropanol, l'éther isopropylique ou leurs mélanges.

5 - Procédé de préparation des énantiomères B(d) et B(l) du Naftidrofuryl, caractérisé en ce que :

a) on sépare le mélange diastéréoisomère Bdl sous forme de l'un de ses sels selon le procédé décrit dans l'une quelconque des revendications 1 à 4.

b) on saponifie dans des conditions non épimérisantes le produit de séparation de l'étape précédente pour obtenir le mélange d'acides (naphtyl-1)-3 tétrahydrofurfuryl-2 propionique B(dl) sous forme cristalline;

c) on salifie le mélange d'acides B(dl) obtenu avec une amine optiquement active;

d) on dédouble le sel d'amine obtenu par recristallisations successives dans des solvants appropriés;

e) on régénère respectivement les acides B(d) et B(l) dédoublés et on les recristallise dans un solvant approprié;

f) on estérifie les acides B(d) et B(l) respectifs avec le N-diéthylamino-2 chloro-1 éthane dans des conditions non épimérisantes; et

g) on salifie les énantiomères B(d) et B(l) obtenus avec des acides pharmaceutiquement acceptables.

6 - Procédé suivant la revendication 5, caractérisé en ce que l'amine optiquement active est l'α-méthylbenzylamine (d) ou (l) respectivement.

7 - Procédé suivant la revendication 5, caractérisé en ce que le solvant de recristallisation de l'étape (d) est l'éther isopropylique.

8 - Procédé suivant la revendication 5, caractérisé en ce que l'acide utilisé dans l'étape (g) est choisi parmi l'acide oxalique, citrique, nitrique, méthanesulfonique.

0069013

Office européen des brevets

# RAPPORT DE RECHERCHE EUROPEENNE

Numéro de la demande

EP 82 40 1155

| Catégorie | DOCUMENTS CONSIDERES COMME PERTINENTS<br>Citation du document avec indication, en cas de besoin, des parties pertinentes | Revendication concernée | CLASSEMENT DE LA DEMANDE (Int. Cl. 3) |
|---|---|---|---|
| X | J. MARCH: "Advanced organic chemistry: Reactions, mechanisms, and structure", 1968, pages 86-87, 92-93, McGraw-Hill Book Company, New York, USA<br>--- | 1-13 | C 07 D 307/16<br>C 07 B 19/00 |
| X | FR-M- 3 843 (LIPHA)<br>* En entier *<br>----- | 1-13 | |
| | | | DOMAINES TECHNIQUES RECHERCHES (Int. Cl. 3) |
| | | | C 07 D 307/00<br>C 07 B 19/00 |

Le présent rapport de recherche a été établi pour toutes les revendications

| Lieu de la recherche | Date d'achèvement de la recherche | Examinateur |
|---|---|---|
| LA HAYE | 10-09-1982 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503.03.82